# FASCICULE DE BREVET EUROPEEN

(11) **EP 1 680 389 B1**
(45) Date de publication et mention de la délivrance du brevet: **21.02.2007**
(21) Numéro de dépôt: 04791448.6
(22) Date de dépôt: 01.10.2004
(51) Int. Cl.: C07C 57/04, C07C 57/07

(54) **PROCEDE DE PURIFICATION DE L'ACIDE (METH)ACRYLIQUE OBTENU PAR OXYDATION D'UN SUBSTRAT GAZEUX**
VERFAHREN ZUR AUFREINIGUNG VON DURCH OXIDIEREN EINES GASFÖRMIGEN SUBSTRATS ERHALTENER (METH)ACRYLSÄURE
METHOD FOR PURIFYING (METH)ACRYLIC ACID OBTAINED BY OXIDIZING A CASEOUS SUBSTRATE

(30) Priorité: 04.11.2003 FR 0312906
(43) Date de publication de la demande: 19.07.2006
(73) Titulaire: Arkema France, 92800 Puteaux (FR)
(72) Inventeur: FAUCONET, Michel, 57730 Valmont (FR); LAURENT, Denis, 57500 Saint-Avold (FR)
(74) Mandataire: Bonnel, Claudine
(86) Numéro de dépôt international: PCT/FR2004/002481
(87) Numéro de publication internationale: WO 2005/054171

(56) Documents cités:
- EP-A- 0 784 046
- EP-A- 1 125 912
- FR-A1- 2 196 986

## Description

La présente invention s'inscrit dans le cadre d' un procédé de fabrication de l'acide (méth) acrylique suivant lequel on conduit l'oxydation d'un substrat gazeux (propane et/ou propylène et/ou acroléine dans le cas de l'acide acrylique ; isobutane et/ou isobutène et/ou alcool tertio-butylique et/ou méthacroléine dans le cas de l'acide méthacrylique) par voie catalytique ou par voie redox, et on effectue la récupération de l'acide (méth)acrylique, à partir du mélange gazeux réactionnel chaud, par absorption à contre-courant d'un solvant.

Les procédés décrits d'absorption de l'acide (méth)acrylique utilisent comme solvant d'absorption soit l'eau, soit un solvant organique, qui est le plus souvent un composé hydrophobe ou un mélange de composés hydrophobe à température d'ébullition nettement plus élevée que l'acide (méth)acrylique.

Les procédés d'absorption à l'aide d' eau fournissent une solution aqueuse d'acide (méth) acrylique qui nécessite des étapes de purification nombreuses et coûteuses pour l'obtention d'acide (méth)acrylique pur.

Au contraire, les procédés d'absorption à l'aide de solvants organiques hydrophobes présentent l'avantage sur les procédés aqueux de réduire le nombre d'étapes de purification nécessaires pour l'obtention d' acide (méth)acrylique pur. Dans ces procédés, on retrouve classiquement les étapes successives d'absorption, strippage, élimination des légers, puis distillation finale de l'acide acrylique pur.

La présente invention relate la récupération sensiblement quantitative (rendement de récupération > 98,5%), en seulement trois étapes, d'acide (méth)acrylique suffisamment débarrassé de ses impuretés légères pour éviter une étape supplémentaire d'étêtage. Un deuxième objectif de l'invention est de réaliser cette récupération sans dilution du gaz résiduel incondensé par un gaz extérieur ajouté à l'étape de strippage, de façon à diminuer la taille de la colonne et la perte de réactifs non consommés dans le gaz résiduel qui doit être purgé. Le troisième objectif est la récupération de l'acide (méth)acrylique pur sans rejet aqueux liquide polluant difficile à éliminer.

Le principal procédé de synthèse de l'acide acrylique utilise une réaction d'oxydation catalytique du propylène à l'aide d'un mélange contenant de l'oxygène. Cette réaction est conduite généralement en phase vapeur, le plus souvent en deux étapes, qui peuvent être conduites dans deux réacteurs distincts ou un seul réacteur :
- la première étape réalise l'oxydation sensiblement quantitative du propylène en un mélange riche en acroléine, dans lequel l'acide acrylique est minoritaire ;
- la deuxième étape complète la conversion de l'acroléine en acide acrylique.

Le mélange gazeux issu de la deuxième étape d'oxydation est constitué :
- d'acide acrylique ;
- de composés légers incondensables dans les conditions de température et de pression habituellement mises en oeuvre (azote, oxygène et propylène non convertis, propane présent dans le propylène réactif, monoxyde et dioxyde de carbone formés en faible quantité par oxydation ultime) ;
- de composés légers condensables, en particulier l'eau, générée par la réaction d'oxydation du propylène, l'acroléine non convertie, des aldéhydes légers, comme le formaldéhyde et l'acétaldéhyde, et l'acide acétique, principale impureté générée dans la section de réaction ;
- de composés lourds : furfuraldéhyde, benzaldéhyde, anyhydride maléique, etc.

Le procédé de synthèse de l'acide méthacrylique par oxydation est dans son principe identique à celui de l'acide acrylique, si ce n'est le substrat réactif (qui peut être l'isobutène ou le tertiobutanol), le produit d'oxydation intermédiaire (méthacroléine) et la nature des composés légers condensables sous-produits (le mélange gazeux réactionnel contient de l'acide acrylique en plus des légers présents dans le gaz réactionnel du procédé de synthèse de l'acide acrylique).

Le second stade de la fabrication consiste à récupérer l'acide acrylique à partir du mélange gazeux chaud, préalablement refroidi à une température de 150-200°C, en introduisant ce gaz en pied d'une colonne d'absorption où il rencontre à contre-courant un solvant introduit en tête de colonne et à l'intérieur de laquelle sont mis en jeu simultanément des processus de refroidissement, condensation, absorption et rectification.

Dans la plupart des procédés décrits, le solvant mis en oeuvre dans cette colonne est l'eau ou un solvant hydrophobe à haut point d'ébullition.

Quel que soit le solvant utilisé, les procédés connus mettent généralement en jeu :
- une colonne d'absorption, alimentée en tête par le solvant, en fond de laquelle est introduit le mélange gazeux réactionnel, qui comprend une section inférieure de refroidissement, et dans laquelle le flux gazeux ascendant subit une condensation partielle en rencontrant un mélange liquide descendant généralement refroidi à travers un échangeur de chaleur, et une section supérieure dont le but est d'absorber dans le solvant le maximum d'acide acrylique ;
- une colonne de désorption, alimentée par le flux de pied de la colonne d'absorption. Le rôle de cette colonne est d'éliminer sélectivement l'essentiel des composés légers absorbés dans l'étape précédente, en particulier l'acroléine non transformée lors de l'étape réactionnelle.

Les composés incondensés plus légers provenant du gaz réactionnel sont éliminés en tête de colonne d'absorption.

De façon à récupérer une partie des réactifs non convertis présents dans ce flux, comme le propylène et l'acroléine, dans le cas d'un procédé de synthèse d'acide acrylique, ou l'isobutène et la méthacroléine dans le cas d'un procédé de fabrication d'acide méthacrylique, une partie du flux gazeux non condensé en tête de colonne d'absorption est généralement recyclée à l'étape de réaction, le reste étant purgé pour éviter l'accumulation des sous-produits dans la boucle de gaz ainsi constituée.

La part maximale de gaz incondensé recyclé à la réaction se trouve également limitée par des critères économiques : la quantité de gaz alimentant les réacteurs étant limitée par les performances des catalyseurs, la productivité d'acide (méth)acrylique est diminuée par la dilution du substrat réactif.

L'acide (méth)acrylique étant un produit sensible à la polymérisation favorisée par les températures élevées, on limite généralement la température de fonctionnement dans la colonne de désorption, soit en réalisant cette distillation à la température du mélange sous une pression réduite, soit en introduisant un gaz inerte en fond d'une colonne de stripping travaillant sous pression atmosphérique ou sous pression réduite, les deux procédés contribuant à diminuer la pression de vapeur des composés condensables, et par conséquent la température des équilibres liquide-gaz régissant la séparation.

Dans le cas de procédés d'absorption utilisant l'eau comme solvant absorbant, le mélange d'acide (méth)acrylique brut récupéré en pied de colonne de désorption contient une part importante d'eau, de l'ordre de 30-50% en poids. Certains composés polaires présentant une forte affinité avec ce solvant, comme les acides carboxyliques, sont absorbés dans l'eau. C'est notamment le cas de l'acide acétique (cas de la synthèse de l'acide acrylique), ou de l'acide acétique et de l'acide acrylique (cas de la synthèse de l'acide méthacrylique), formés par réaction secondaire lors de l'étape de réaction, qui se retrouve totalement dans ce flux d'acide (méth)acrylique brut.

La séparation des impuretés majoritaires, à savoir l'eau et de l'acide acétique (cas de la synthèse de l'acide acrylique) ou l'eau, l'acide acétique et l'acide acrylique (cas de la synthèse de l'acide méthacrylique), dans le but d'obtenir respectivement de l'acide acrylique pur ou de l'acide méthacrylique pur, sont difficiles. Elles nécessitent un grand nombre de colonnes de séparation. L'étape de déshydratation est généralement réalisée en présence de solvant non miscible à l'eau, dans une colonne d'extraction ou de distillation hétéroazéotropique, couplée généralement à une colonne de récupération du solvant solubilisé partiellement dans la phase aqueuse extraite, en vue de le recycler en amont du procédé. L'étape d'élimination des légers (étêtage) met en jeu habituellement une à deux colonnes et l'étape de séparation des lourds (équeutage) est réalisée dans une ultime colonne, de laquelle est extrait l'acide (méth)acrylique pur en tête.

Les procédés d'absorption par solvant non aqueux présentent l'avantage de réduire les étapes de purification, notamment en évitant le recours à des procédés lourds et coûteux de séparation de l'eau, l'utilisation de solvant hydrophobes permettant de réaliser l'élimination de l'eau en tête de colonne d'absorption.

Un autre avantage des procédés décrits d'absorption par cette voie, par rapport à la voie d'absorption par l'eau, est de faciliter l'élimination des composés légers.

Le rôle de la colonne de désorption située en aval de la colonne d'absorption est de réduire les teneurs de composés condensables légers dans le flux de pied de colonne d'absorption, en particulier l'acroléine non convertie, l'eau résiduelle et l'acide acétique (cas de la synthèse de l'acide acrylique) ou la méthacroléine, l'eau, l'acide acrylique et l'acide acétique (cas de l'acide méthacrylique).

Cette élimination des impuretés légères n'est toutefois pas totale, dans les procédés décrits dans l'état antérieur de la technique. Ainsi, le procédé de purification d'acide acrylique avec absorption par un mélange de diphényle et de diphényléther, décrit dans le brevet français FR-B-2 146 386, aboutit à un acide acrylique brut titrant encore 0,5% en poids d'acide acétique et 0,5% en poids d'eau. Le brevet américain US-A-5 426 221, décrivant un procédé avec absorption de l'acide acrylique par un mélange de diphényle, diphényléther et phtalate de diméthyle, permet d'améliorer l'élimination d'eau (représentant 0,04% en poids dans l'acide acrylique brut distillé selon l'exemple), mais il reste encore 0,26% en poids d'acide acétique dans l'acide acrylique purifié sans étape supplémentaire d'étêtage. Le procédé d'absorption par des esters carboxyliques décrit dans le brevet français FR-B-2 196 986 permet d'obtenir une qualité d'acide acrylique titrant encore 0,3% en poids d'acide acétique et 0,2% en poids d'eau.

Les qualités d'acide acrylique obtenues par ce procédé sont insuffisantes, en l'absence de purification complémentaire, pour l'utilisation du monomère dans ses applications classiques. Pour parfaire l'élimination des composés légers sans rajouter de colonne supplémentaire, des solutions ont été proposées consistant à réaliser l'étêtage dans une section supérieure ajoutée à la colonne de distillation finale de l'acide acrylique. Ainsi, il est fait mention dans le brevet européen EP-B-706 986 d'une colonne de récupération où l'acide acrylique est obtenu en soutirage latéral, la section supérieure de la colonne permettant de concentrer les légers résiduels en tête, en vue de les éliminer. L'inconvénient majeur d'un tel procédé est la difficulté de séparation des légers, en particulier l'acide acétique, qui oblige à augmenter de façon sensible le nombre de plateaux de la colonne et le débit de flux condensé renvoyé en tête (reflux) pour assurer la séparation et réduire la perte d'acide acrylique. Il en résulte une augmentation sensible de la taille de la colonne, et donc du coût d'investissement, où, à taille égale de colonne, une diminution de la capacité de distillation de la colonne. En outre, ce système génère un flux contenant encore de l'AA qui, pour éviter sa perte, doit être recyclé à une étape précédente. Il en résulte alors un accroissement de la complexité du procédé et une limitation de la capacité de la colonne recevant ce flux.

Un autre inconvénient des procédés d'absorption par solvants lourds hydrophobes est le fait que de grandes quantités de solvant sont nécessaires pour absorber la totalité de l'acide acrylique présent dans les gaz de réaction. Dans le brevet français FR-B-2 146 386, pour atteindre un taux de récupération suffisant et éviter les pertes coûteuses d'acide acrylique non absorbé, le rapport massique de solvant (mélange de diphényle et diphényléther) par rapport à l'acide acrylique est d'environ 9/1, soit une concentration d'acide acrylique dans le mélange brut, après absorption et désorption, de l'ordre de 10%. Les conséquences sont la taille importante des colonnes et des équipements et stockages annexes, l'augmentation du coût énergétique lié à la vaporisation dans les colonnes de solvant à forte température d'ébullition.

Par ailleurs, les procédés d'absorption par solvants non aqueux décrits dans la littérature ont en commun la particularité de réaliser l'étape de désorption par strippage par un gaz inerte introduit en fond de colonne. Ce gaz inerte peut être de l'azote, de l'air ou tout ou partie des gaz incondensés en tête de colonne d'absorption. La quantité introduite représente généralement entre 15% et 30% de l'ensemble des gaz entrants provenant de l'étape de réaction. L'inconvénient de cette introduction de gaz extérieur est que, étant renvoyé dans la colonne d'absorption pour récupérer l'acide acrylique qu'il contient, il vient s'ajouter au gaz de réaction qui en constitue l'alimentation principale, et par conséquent la capacité maximale d'absorption de la colonne s'en trouve limitée. En définitive, pour une même capacité de production de l'atelier, la taille des colonnes d'absorption et de strippage augmente avec le débit de gaz externe introduit.

Un autre inconvénient du procédé de strippage par un gaz externe est de diluer les gaz incondensés en tête de colonne et de provoquer par conséquent une dilution de la partie de ces gaz renvoyés à l'étape de réaction dans le but de récupérer les composés nobles qu'ils contiennent (propylène, acroléine, traces d'acide acrylique). Ainsi, pour une même quantité de composés nobles recyclés en l'absence de gaz extérieur de strippage, le débit des gaz entrant dans le réacteur se voit augmenter par l'introduction de ce gaz extérieur. La conséquence est soit une augmentation du débit de gaz alimentant les réacteurs, à débit de réactifs constants, qui se traduirait par une accélération du vieillissement des catalyseurs causée par une température de travail plus élevée pour atteindre les mêmes performances de rendement, soit, à débit constant de gaz entrant dans les réacteurs, une réduction de la productivité par diminution du débit des réactifs.

Le brevet européen EP 706 986, qui décrit un procédé d'absorption par solvant non aqueux sans colonne de désorption, ne permet pas d'atteindre les performances d'élimination de composés légers, en particulier d'acide acétique, revendiqués dans les procédés décrits avec colonne de désorption.

Enfin, les brevets décrits dans l'art antérieur présentent une section de condensation de l'eau en tête de colonne d'absorption. Le flux aqueux condensé est particulièrement riche en composés polaires présentant des volatilités plus faibles ou proches celle de l'eau, en particulier en acides organiques comme l'acide formique, l'acide acétique ou l'acide acrylique. Du fait du caractère polluant de ce flux, celui-ci ne peut être éliminé sans traitement ultérieur d'élimination, généralement par incinération des composés organiques. Le coût de ce traitement d'incinération est augmenté par le fait que le flux à traiter est un flux liquide, constitué principalement d'eau.

Par la demande de brevet européen EP-A2-1 125 912, on connaît un procédé de purification de l'acide acrylique comportant une étape d'absorption par un solvant lourd hydrophobe, suivie d'une étape de distillation dans une colonne sous pression réduite pour étêtage, en faisant suivre par une distillation pour obtenir l'acide acrylique sans le solvant.

Conformément à ce procédé, le débit massique du solvant lourd est de 0,2-4,5 fois le débit massique de l'acide acrylique et le flux adressé à la colonne sous pression réduite contient de l'acide acrylique à raison de 18 à 75% en poids.

La Société déposante a recherché à améliorer encore le rendement d'absorption et à diminuer le taux d'acide acétique dans l'acide acrylique.

Cet objectif a été atteint par le choix d'une plage particulière du rapport solvant/acide (méth)acrylique et par la réalisation de l'étape de désorption à l'aide d'une colonne de rectification qui se distingue de la colonne de distillation de la demande européenne EP-A2-1 125 912 par le fait qu'elle fonctionne avec alimentation en tête, sans reflux, et par des conditions particulières de fonctionnement de cette colonne, telles que son débit de tête rapporté au débit d'acide (méth)acrylique introduit dans la colonne d'absorption.

Dans le procédé selon la demande de brevet européen EP-A2-1 125 912, les deux colonnes d'absorption et de distillation sont indépendantes. Du fait de son fonctionnement avec un taux de reflux imposé important (de préférence 3/1 à 6/1), le débit de tête de la colonne de distillation renvoyé dans la colonne d'absorption, rapporté au débit d'acide acrylique circulant dans cette dernière colonne, est très faible (< 0,1/1). Au contraire, selon la présente invention, les deux colonnes d'absorption et de rectification sans reflux forment un ensemble indissociable, les performances de l'une étant dépendantes de celles de l'autre, et la composition du flux de la boucle circulant entre le fond de C1 et la tête de C2 permet d'améliorer les performances de séparation de l'ensemble.

De façon surprenante, les performances de l'ensemble sont améliorées par rapport au procédé selon EP-A2-1 125 912, et ce, malgré des efficacités de colonnes plus faibles : la colonne d'absorption a été estimée à 65-70 plateaux théoriques dans les exemples de la demande de brevet précitée, contre 35-40 dans les exemples de l'invention ; et la colonne de distillation est de 28 plateaux Oldershaw dans les exemples de la demande de brevet européen précitée contre 15 plateaux perforés à déversoirs d'efficacité théorique équivalente dans les exemples de la présente invention.

En outre, l'utilisation d'une colonne de rectification sans reflux, alimentée en tête, présente l'avantage, par rapport à une colonne de rectification avec reflux, de réduire significativement la formation de polymères en tête de colonne, la concentration d'acide (méth)acrylique dans le liquide présent sur les plateaux supérieurs étant nettement plus faible que dans une colonne de rectification.

Le procédé dans la présente invention permet de réaliser, avec seulement deux colonnes (une colonne d'absorption, une colonne de rectification) à la fois :
- la récupération de l'acide (méth)acrylique avec un rendement supérieur à 98,5%, avantageusement supérieur à 99% ;
- la récupération d'un flux d'acide (méth)acrylique brut concentré dans le solvant, contenant au moins 20% d'acide (méth)acrylique ;
- l'élimination quasi totale des composés légers, en particulier de l'eau et de l'acide acétique (cas de la synthèse d'acide acrylique) ou de l'eau, de l'acide acétique et de l'acide acrylique (cas de la synthèse de l'acide méthacrylique), permettant d'obtenir, sans étape d'étêtage supplémentaire, un acide (méth)acrylique contenant avantageusement moins de 0,05% d'eau, moins de 0,01% d'acroléine et moins de 0,1% d'acide acétique.

Le pourcentage d'acide (méth)acrylique obtenu dans le pied de la colonne de rectification est notamment de 10-33% en poids, en particulier de 20% à 25% en poids.

Par ailleurs, l'étape de rectification étant réalisée sans introduction de gaz inerte extérieur, le procédé décrit a l'avantage supplémentaire de réduire la taille des équipements de purification et de faciliter le recyclage à l'étape de réaction des gaz incondensés en tête de colonne d'absorption.

Enfin, selon un mode de réalisation particulièrement avantageux, le gaz incondensé en tête de colonne d'absorption ne subit pas de condensation, et est envoyé sous forme gazeuse, pour partie à l'étape de réaction et pour partie dans une section de traitement de purge (incinération). Il en résulte une diminution substantielle des rejets aqueux polluants et du coût de traitement du flux de purge.

La présente invention a donc pour objet un procédé de purification de l'acide (méth)acrylique obtenu par oxydation par voie catalytique ou par voie redox d'un substrat gazeux constitué par du propane et/ou du propylène et/ou de l'acroléine dans le cas de la fabrication de l'acide acrylique, et par de l'isobutane et/ou de l'isobutène et/ou de l'alcool tertiobutylique et/ou de la méthacroléine dans le cas de la fabrication de l'acide méthacrylique, ledit mélange gazeux (1) étant principalement constitué par :
- du propane et/ou du propylène ou de l'isobutane et/ou de l'isobutène suivant que le substrat en comportait ;
- des produits d'oxydation ultime ;
- l'acide (méth)acrylique recherché ;
- de la (méth)acroléine ;
- de l'alcool tertiobutylique dans le cas de la fabrication l'acide méthacrylique ;
- de la vapeur d'eau ;
- de l'acide acétique avec, dans le cas de la fabrication de l'acide méthacrylique de l'acide acrylique comme sous-produit ; et
- des produits lourds de réactions secondaires,
suivant lequel on adresse le mélange gazeux de réaction (1) en pied d'une colonne d'absorption (C1) laquelle est alimentée en tête et à contre-courant par au moins un solvant lourd d'absorption hydrophobe, pour obtenir :
- en tête de la colonne (C1), un flux gazeux (7) constitué par :
   - le propane et/ou le propylène ou l'isobutane et/ou l'isobutène, suivant que l'on fabrique l'acide acrylique ou l'acide méthacrylique, et les produits d'oxydation ultime du mélange (1),
   - des quantités majeures d'eau et d'acide acétique dans le cas de la fabrication de l'acide acrylique, ou d'eau, d'acide acétique et d'acide acrylique dans le cas où l'on fabrique l'acide (méth)acrylique ; et
   - la (métha)croléine ;
- en pied de ladite colonne (C1), un flux (4) constitué par :
   - l'acide (méth)acrylique ;
   - le ou les solvants lourds d'absorption ;
   - les produits lourds de réactions secondaires ; et
   - des quantités mineures d'acide acétique et d'eau, dans le cas de la fabrication de l'acide acrylique et d'acide acétique, d'acide acrylique et d'eau dans le cas de la fabrication de l'acide méthacrylique.
   puis on adresse le flux issu de la colonne (C1) à une colonne de séparation (C2) dans laquelle on conduit une séparation pour obtenir :
- en tête, un flux constitué par les impuretés légères que l'on renvoie à la partie inférieure de la colonne d'absorption (C1) ; et
- en pied, un flux constitué par :
   - l'acide (méth)acrylique en solution dans le ou les solvants d'absorption ;
   - une faible proportion d'acide acétique dans le cas de la fabrication de l'acide acrylique et d'acide acétique et d'acide acrylique dans le cas de la fabrication de l'acide méthacrylique ;
   - les produits lourds des réactions secondaires ; et
   - le ou les inhibiteurs de polymérisation,
caractérisé par le fait que l'on fait fonctionner la colonne (C1) avec un débit du solvant lourd qui est de 3 à 5,6 fois le débit de l'acide (méth)acrylique dans le mélange gazeux d'alimentation, et par le fait que l'on utilise, comme colonne de séparation (C2), une colonne de rectification que l'on fait fonctionner avec une alimentation en tête et sans reflux.

Conformément à une caractéristique intéressante du procédé selon l'invention, on fait fonctionner la colonne (C2) dans des conditions telles que son débit de tête rapporté au débit d'acide (méth)acrylique introduit dans la colonne d'absorption (C1) est compris entre 0,5/1 et 4/1, notamment entre 2/1 et 3/1.

On utilise avantageusement une colonne d'absorption (C1) comportant :
- dans sa partie inférieure, au moins une section de refroidissement (S1) équipée d'un système de recirculation, à travers un échangeur externe (E1) d'une partie du flux recueilli en partie inférieure de ladite ou desdites sections (S1) pour le renvoyer en tête desdites sections ; et
- dans sa partie supérieure, une section (S2) d'absorption et de rectification du mélange gazeux de réaction.

On utilise notamment une section (S2) dont le nombre de plateaux théoriques est de 25 à 50, de préférence de 30 à 45.

On conduit l'absorption dans la colonne (C1) généralement à la pression atmosphérique ou sous une pression proche de la pression atmosphérique et avantageusement à une température d'introduction du ou des solvants de 20 à 80°C, de préférence de 30 à 60°C.

Conformément à des caractéristiques particulières du procédé selon l'invention on fait fonctionner la colonne (C1) à une température de pied de 50 à 120°C, notamment de 70 à 100°C ; à une température des gaz en tête de 40 à 70°C, notamment de 50 à 60°C ; et on introduit les gaz de réaction à une température de 100°C à 200°C, notamment de 130°C à 180°C.

La littérature brevets donne de nombreux exemples de solvants lourds hydrophobes. On utilise avantageusement un ou des solvants lourds d'absorption hydrophobes ayant un point d'ébullition sous pression atmosphérique supérieur à 200°C, le ditolyléther étant particulièrement préféré comme solvant lourd hydrophobe. On peut alimenter la colonne d'absorption (C1) par un ou des solvants purs et/ou par un ou des solvants provenant d'un recyclage d'un ou des flux obtenus dans des étapes ultérieures de purification.

On conduit généralement l'absorption dans la colonne (C1) en présence d'au moins un inhibiteur de polymérisation, choisi notamment parmi les dérivés phénoliques comme l'hydroquinone et ses dérivés tels que l'éther méthylique de l'hydroquinone, la phénothiazine et ses dérivés, tels que le bleu de méthylène, les quinones, telles que la benzoquinone, les thiocarbamates métalliques, tels que le dibutyldithiocarbamate de cuivre, les composés à groupements nitroso, tels que la N-nitroso-phénylhydroxylamine, les amines telles que les dérivés de la paraphénylènediamine, ou les composés N-oxyls, comme le 4-hydroxy-2,2,6,6-tétraméthyl-pipéridine-N-oxyl.

Conformément à un premier mode de réalisation du procédé selon l'invention, on évacue le flux gazeux issu de la tête de la colonne (C1), pour partie à la section de réaction, pour partie à une étape d'incinération ou de purge.

Conformément à un second mode de réalisation du procédé selon l'invention, on envoie le flux gazeux issu de la tête de colonne (C1) en pied d'une section de condensation (S3) où ce mélange gazeux est mis en contact intime avec un courant liquide descendant alimenté en tête de ladite section (S3) et constitué par le recyclage d'une partie du flux de pied de ladite section (S3) préalablement refroidie par un échangeur de chaleur externe (E4).

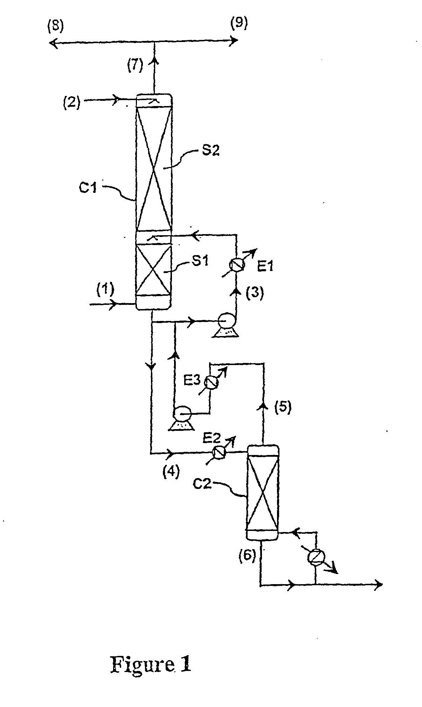

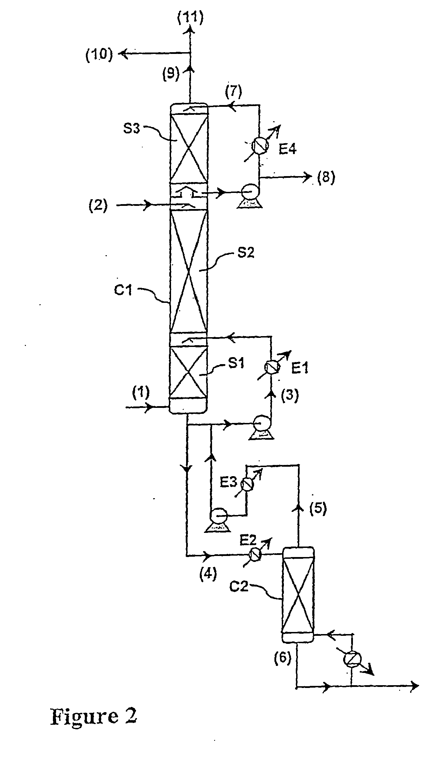

Les schémas joints en annexe permettent d'illustrer l'invention (Figures 1 et 2).

La Figure 1 décrit un premier mode de réalisation. Le mélange gazeux de réaction issu de l'oxydation du propylène et de l'acroléine (dans le cas de la fabrication de l'acide acrylique) ou de l'oxydation de l'isobutène et de la méthacroléine (dans le cas de la fabrication de l'acide méthacrylique), principalement constitué :
- d'une part, de composés incondensables dans les conditions de pression de fonctionnement de la colonne : propylène, produits d'oxydation ultimes (CO, CO₂) ;
- d'autre part, de composés condensables : acide acrylique, acroléine, eau et acide acétique dans le cas de la fabrication de l'acide acrylique, ou acide méthacrylique, méthacroléine, eau, acide acrylique et acide acétique dans la cas de la fabrication de l'acide méthacrylique, produits de réactions secondaires plus lourds en quantités très faibles,
est envoyé (flux 1) en pied d'une colonne C1 d'absorption alimentée en tête et à contre-courant par un solvant (flux 2 : solvant hydrophobe lourd à température d'ébullition supérieure à 200°C sous pression atmosphérique).

De manière préférentielle, la colonne comporte :
- dans sa partie inférieure une ou plusieurs sections de refroidissement S1 équipées de systèmes de recirculation, à travers un ou plusieurs échangeurs externes E1, d'une partie du flux recueilli en partie inférieure de S1 (flux 3), pour le renvoyer en tête de cette section ;
- dans sa partie supérieure une section S2 dans laquelle s'opère l'absorption et la rectification du mélange.

L'alimentation de solvant est réalisée au-dessus de la section S2. Le solvant introduit pourra être un solvant pur ou provenir d'un recyclage de flux obtenu dans les étapes ultérieures de purification. De préférence, la colonne C1 fonctionne sous une pression proche de la pression atmosphérique.

Le flux 4 obtenu en pied de colonne C1 est principalement composé de l'acide acrylique et du solvant, ainsi que de faibles quantités d'acide acétique, d'eau et d' acroléine. Ce flux 4, dont la température peut éventuellement être ajustée à travers un échangeur E2, est ensuite débarrassé de ces impuretés légères (étape de désorption) en l'envoyant en tête d'une colonne C2 de distillation dans laquelle elles seront concentrées en tête, en mélange avec de l'acide acrylique et des traces de solvant. Le flux gazeux 5 est condensé à travers un échangeur E3 et renvoyé sur la colonne C1, en un endroit situé dans la partie inférieure de celle-ci, de préférence dans l'une des boucles de refroidissement S1. Le flux 6 obtenu en pied de colonne C2 est alors constitué principalement de l'acide acrylique en solution dans le solvant, ainsi que des impuretés lourdes, issues de réactions secondaires, présentes en faibles quantités dans le flux de gaz de réaction.

De manière avantageuse, la colonne C2 fonctionne sous pression réduite et le gaz utilisé pour la désorption des composés légers est généré par l'ébullition du mélange de fond de colonne à travers un échangeur (bouilleur).

Le flux gazeux 7 issu de la colonne C1 contient les composés initialement présents dans le gaz de réaction et non absorbés : produits incondensables à la pression de fonctionnement de la colonne (propylène, CO, CO₂ dans le cas de la fabrication de l'acide acrylique ; isobutène, CO, CO₂, dans le cas de la fabrication de l'acide méthacrylique), eau, acroléine ou méthacroléine suivant que l'on fabrique l'acide acrylique ou l'acide méthacrylique, acide acétique dans le cas de la fabrication de l'acide acrylique ou acide acétique et acide acrylique dans le cas de la fabrication de l'acide méthacrylique.

Selon le premier mode de réalisation (Figure 1), le flux gazeux 7 issu de la tête de colonne C1 contient la totalité de l'eau, de l'acide formique, de l'acide acétique et de l'acroléine, qui sont évacués directement, pour partie à la section réaction (flux 8), en vue de compléter la conversion des réactifs nobles qu'il contient, et pour partie (flux 9) à une étape de traitement d'incinération (purge).

Selon un deuxième mode de réalisation (Figure 2), le flux gazeux ascendant issu de la section S2 de la colonne C1 est envoyé en pied d'une section de condensation S3 où ce mélange gazeux est mis en contact intime avec un courant liquide descendant (flux 7), alimenté en tête de cette section, et constitué par le recyclage d'une partie du flux de pied de section S3 préalablement refroidi par un échangeur E4.

La section S3 peut aussi être constituée d'une colonne distincte en série avec la colonne C1. Le flux gazeux 9 de tête de section S3 contient les composés présents dans le flux gazeux issu de la tête de section S2, sauf une partie de l'eau et la totalité de l'acide formique, de l'acide acétique (cas de la fabrication de l'acide acrylique) ou des acides acétique et acrylique (cas de la fabrication de l'acide méthacrylique), qui sont éliminés dans le flux 8. L'essentiel du flux 9 sera avantageusement recyclé à l'étape de réaction (flux 10), pour convertir les réactifs nobles qu'il contient, et on pourra réaliser une faible purge de ce flux (flux 11) pour éviter l'accumulation dans la boucle ainsi constituée des composés incondensables résultant de l'oxydation ultime du propylène (CO, CO2) et de l'azote provenant de l'air introduit au niveau de l'étape de réaction.

### EXEMPLES

Dans ces Exemples, les abréviations suivantes ont été utilisées :
- AA : acide acrylique ;
- AcOH : acide acétique ;
- ACO : acroléine ;
- DTE : ditolyléther ;
- EMHQ : éther méthylique de l'hydroquinone

Les exemples décrits ci-après viennent illustrer l'invention. Les pourcentages sont indiqués en pourcentages massiques.

### Exemple 1

Le montage expérimental utilisé est celui qui est représenté sur la Figure 1 du dessin annexé.

Le mélange gazeux alimentant la colonne d'absorption C1 est représentatif d'un milieu réactionnel issu du deuxième étage d'un réacteur d'oxydation du propylène en acide acrylique. Il est constitué de composés condensables :
- AA : 14,1% ;
- AcOH : 0,8% ;
- ACO : 0,45% ;
- H₂O : 7,81%
et de composés inertes incondensables :
- N₂ : 69,83% ;
- O₂ : 2,5% ;
- propylène : 1,2% ;
CO₂ : 3,31%.

Ce mélange gazeux est envoyé à un débit de 709 g/h, à une température de 165°C, en pied d'une colonne C1 en verre, ayant une efficacité globale de 42 plateaux théoriques, et constituée d'une section inférieure S1 de refroidissement équipée de 5 plateaux perforés avec déversoir, et d'une section supérieure S2 d'absorption-distillation équipée de 14 éléments de garnissage de type Sulzer EX. L'alimentation en gaz réactionnel se fait en bas de la section inférieure S1 de refroidissement.

La colonne C1 est alimentée en tête de sa section supérieure S2 par un flux constitué de DTE, à un débit de 400 g/h (rapport Solvant/Acide acrylique dans le gaz de réaction = 4/1), dans lequel on a préalablement dissous 0,1% d'EMHQ, en tant qu'inhibiteur de polymérisation. La température d'introduction du solvant d'absorption est de 54°C. La pression de travail dans la colonne C1 est la pression atmosphérique. La température mesurée en pied de colonne C1 est de 89°C. En tête de cette colonne C1, la température est de 52°C.

Pour analyser la totalité des composés organiques entraînés dans le flux gazeux de tête, celui-ci est absorbé dans une colonne d'abattage où il rencontre à contre-courant un flux important d'eau (13700 g/h) à une température de 20°C.

L'acide acétique récupéré en tête représente 98,7% de l'acide acétique initial, et l'acide acrylique, 0,3% de l'acide acrylique initial (rendement de récupération d'acide acrylique : 99,7%).

Une partie du flux liquide obtenu en pied de section S1 de la colonne d'absorption C1 est envoyée grâce à une pompe à travers un échangeur à double enveloppe E1, où il est refroidi à 70°C, en tête de la section S1.

Le reste du mélange obtenu en pied de section S1 est refroidi à travers un échangeur E2, jusqu'à une température de 35°C, puis est envoyé au moyen d'une pompe en tête d'une colonne C2 en verre, équipée de 15 plateaux perforés avec déversoir, pour une efficacité globale de 11 plateaux théoriques. La colonne C2 est munie en pied d'un bouilleur à thermosiphon et en tête d'un condenseur E3.

On opère la distillation dans cette colonne C2 sous pression réduite de 187 hPa (140 mm Hg). La température mesurée en fond de colonne C2 est de 113°C, et la température de tête atteint 88°C.

La totalité des vapeurs condensées dans l'échangeur E3 (318 g/h, soit un rapport massique de débit de tête rapporté au débit d'acide acrylique introduit dans la colonne C1 de 3,2/1) est renvoyée par l'intermédiaire d'une pompe dans la boucle de refroidissement externe de la colonne C1, en amont de la pompe assurant la recirculation du mélange de pied de section S1, à travers l'échangeur E1, vers la tête de cette section.

Le flux liquide extrait en pied de cette colonne C2 (497,6 g/h) titre 20,2% d'acide acrylique. Après distillation pour séparer le solvant, l'acide acrylique obtenu en tête de colonne contient 0,068% d'acide acétique, moins de 0,01% d'acroléine et moins de 0,01% d'eau.

### Exemple 2

Le montage expérimental est identique à celui de l'Exemple 1 précédent.

La colonne C1 fonctionne sous pression atmosphérique, tandis que la pression de travail de la colonne C2 est de 187 hPa (140 mm Hg).

Un mélange gazeux de même composition alimente avec le même débit la colonne C1 dans sa partie basse, à la température de 165°C.

Le débit de DTE introduit à une température de 53°C en tête de colonne C1 est cette fois réduit à 300 g/h (rapport Solvant / Acide acrylique dans le gaz de réaction = 3/1).

La température de tête de colonne est de 51°C, celle de pied, de 77°C. Les températures de tête et de fond de colonne C2 sont de respectivement 83°C et 111°C. La partie du flux de pied de colonne C1 renvoyée en tête de section S1 est refroidie à 68°C à travers l'échangeur E1. Le reste du flux liquide de pied de colonne C1 alimentant la colonne C2 est préalablement refroidi à 34°C. Le rapport massique du débit de tête de la colonne C2 rapporté au débit d'acide acrylique alimentant la colonne C1 est de 3, 3/1.

L'acide acétique et l'acide acrylique récupérés après absorption dans la colonne d'abattage à l'eau (17600 g/h) représentent respectivement 98,6% et 0,9% des composés initiaux (rendement de récupération d'acide acrylique : 99,1%).

La concentration d'acide acrylique dans le flux de pied de colonne C2 atteint 24,5%. Après séparation du solvant par distillation, l'acide acrylique obtenu ne contient que 0,087% d'acide acétique et moins de 0,01% d' acroléine et eau.

### Exemple 3

Dans les mêmes conditions de montage expérimental, pression, température, composition et débit du gaz entrant que l'Exemple 1, l'acide acrylique est absorbé à contre-courant d'un flux de DTE alimenté en tête de colonne à un débit de 560 g/h (rapport Solvant / Acide acrylique dans le gaz de réaction : 5,6/1) et à une température de 50°C.

La température de tête de colonne est de 51,6°C, celle de pied, de 82°C. Les températures de tête et de fond de colonne C2 sont de respectivement 86, 4°C et 120,5°C. La partie du flux de colonne C1 renvoyée en tête de section S1 est refroidie à 69°C à travers l'échangeur E1. Le reste du flux liquide de pied de colonne C1 alimente la colonne C2 à une température de 61°C. Le rapport massique du débit de tête de la colonne C2 rapporté au débit d'acide acrylique alimentant la colonne C1 est de 1,1/1.

Le flux obtenu en pied de colonne C2 contient 15,1% d'acide acrylique. Après séparation du solvant par distillation, la concentration d'acide acétique dans l'acide acrylique obtenu est de 0,058%, et ce flux contient moins de 0,01% d'acroléine et eau.

L'acide acétique et l'acide acrylique récupérés après absorption dans la colonne d'abattage à l'eau (21120 g/h) représentent respectivement 99,55% et 0,4% des composés initiaux (rendement de récupération d'acide acrylique : 99,6%).

### Exemple 4 (comparatif)

Dans les mêmes conditions de montage expérimental, pression, température, composition et débit du gaz entrant que l'Exemple 1, l'acide acrylique est absorbé à contre-courant d'un flux de DTE alimenté en tête de colonne à un débit de 100 g/h (rapport Solvant / Acide acrylique dans le gaz de réaction = 1/1) et une température de 45°C.

La température de tête de colonne est de 52,6°C, celle de pied de 78,9°C. Les températures de tête et de fond de colonne C2 sont de respectivement 84,9°C et 104,4°C. La partie du flux de pied de colonne C1 renvoyée en tête de section S1 est refroidie à 70°C à travers l'échangeur E1. Le reste du flux liquide de pied de colonne C1 alimente la colonne C2 à une température de 74°C. Le rapport massique du débit de tête de la colonne C2 rapporté au débit d'acide acrylique alimentant la colonne C1 est de 3,9/1.

Le flux obtenu en pied de colonne C2 contient 44,6% d'acide acrylique. Après séparation du solvant par distillation, la concentration d'acide acétique dans l'acide acrylique obtenu est de 0,045%, et ce flux contient moins de 0,01% d'acroléine et eau.

L'acide acétique et l'acide acrylique récupérés après absorption dans la colonne d'abattage à l'eau (19550 g/h) représentent respectivement 99,35% et 17,2% des composés initiaux. Le rendement de récupération d'acide acrylique est donc particulièrement faible : 82,2%.

Pour tenter d'améliorer ce rendement de récupération, l'expérience a été renouvelée dans des conditions identiques, hormis le fait que la colonne C1 contenait 3 éléments SULZER EX supplémentaires dans sa section S2. L'efficacité globale de la colonne était alors de 50 plateaux théoriques.

A l'issue de l'essai, les performances ne sont pas significativement améliorées : concentration d'acide acétique dans l'acide acrylique distillé : 0,054%, et rendement de récupération d'acide acrylique : 84%.

Une troisième tentative en vue d'améliorer le rendement de récupération d'acide acrylique, dans les mêmes conditions que décrites ci-avant, mais en réduisant la température d'alimentation du solvant en tête de colonne C1 à 20°C s'est soldée par un échec. Dans les conditions de fonctionnement sous pression atmosphérique de la colonne d'absorption, l'élimination des composés légers en tête de colonne devenait impossible, provoquant ainsi l'accumulation de ces légers en pied de colonne, l'engorgement de la colonne C2 et rapidement la formation de polymères dans cette dernière colonne.

### Exemple 5 (comparatif)

Cet exemple est réalisé dans les mêmes conditions de pression, température, composition et débit du gaz entrant que l'Exemple 1. Le montage est celui de l'Exemple 1, hormis le fait que la colonne d'absorption est équipée de 17 éléments Sulzer EX dans la section S2, soit une efficacité globale de la colonne C1 de 50 plateaux théoriques. L'acide acrylique est absorbé à contre-courant d'un flux de ditolyléther alimenté en tête de colonne à un débit de 233 g/h (rapport Solvant / Acide acrylique dans le gaz de réaction = 2 ,33/1) et une température de 45°C.

La température de tête de colonne C1 est de 51,5°C, celle de pied de 76,9°C. Les températures de tête et de fond de colonne C2 sont de respectivement 83, 3°C et 108,5°C. La partie du flux de pied de colonne C1 renvoyée en tête de section S1 est refroidie à 75°C à travers l'échangeur E1. Le reste du flux liquide de pied de colonne C1 alimente la colonne C2 à une température de 35°C. Le rapport massique du débit de tête de la colonne C2 rapporté au débit d'acide acrylique alimentant la colonne C1 est de 3/1.

Le flux obtenu en pied de colonne C1 contient 29,5% d'acide acrylique. Après séparation du solvant par distillation, la concentration d'acide acétique dans l'acide acrylique obtenu en tête de colonne est de 0,06%, et ce flux contient moins de 0,01% d'acroléine et eau.

L'acide acétique et l'acide acrylique récupérés après absorption dans la colonne d'abattage à l'eau (18540 g/h) représentent respectivement 99,04% et 3% des composés initiaux. Le rendement de récupération d'acide acrylique reste donc faible : 97%, malgré l'augmentation de l'efficacité de la colonne C1.

### Exemple 6

Le principe du procédé mis en jeu dans ce troisième exemple est celui de la Figure 2 en annexe. Les colonnes C1 et C2 sont identiques, fonctionnent aux mêmes pressions que dans les exemples précédents, et une colonne supplémentaire de condensation partielle C3 (équivalente à la section S3 de la Figure 2), travaillant sous pression atmosphérique, est placée en série en tête de la colonne C1, de façon à éliminer les composés organiques entraînés dans le flux gazeux de tête de cette colonne, sous forme d'un flux aqueux.

Le mélange gazeux réactionnel (709 g/h) de même composition que dans les exemples précédents est introduit à la température de 164°C dans la colonne C1. Le DTE alimente cette même colonne C1 en tête, à un débit de 300 g/h, à la température de 53°C. Les températures respectives de tête et de pied de colonne C1 sont de 51°C et 77°C, celles de colonne C2 de 83°C (tête) et 111°C (pied). Le flux de pied de colonne C1 dans la boucle de recirculation en tête de section S1 est refroidi à 68°C à travers l'échangeur E1. Le reste de ce flux de pied de colonne C1 alimente la colonne C2 à une température de 32°C. Le rapport massique du débit de tête de la colonne C2 rapporté au débit d'acide acrylique alimentant la colonne C1 est de 1,9/1.

Le flux liquide obtenu en pied de colonne C2 (acide acrylique brut) contient 24,5% d'acide acrylique. Après distillation en vue d'éliminer le solvant, l'acide acrylique pur obtenu titre 0,08% d'acide acétique, moins de 0,01% d'eau et moins de 0,01% d'acroléine.

Le flux gazeux extrait de la tête de colonne C2 est envoyé en pied de la colonne de condensation C3, où il rencontre à contre-courant un flux aqueux constitué par la recirculation, en tête de la colonne S3, et à travers un échangeur E4, d'une partie du flux récupéré en pied de cette colonne. La température des gaz incondensés en tête de cette section est de 42°C. Une purge de 22,3 g/h (soit 35% de l'eau présente dans le gaz réactionnel initial) est réalisée au niveau de la boucle de recirculation de cette colonne. Ce mélange aqueux purgé titre 11,24% d'acide acétique (soit 49% de l'acide acétique initial) et 0,08% d'acroléine (soit 0,56% de l'acroléine initiale).

Le flux gazeux sortant en tête de colonne de condensation est envoyé à contre-courant d'un flux important d'eau (18250 g/h) dans une colonne d'abattage destinée à quantifier les pertes de produits organiques dans le flux incondensé en tête de S3. La totalité de l'acide acétique et de l'acide acrylique récupérés en tête de colonne C2 (colonne de condensation et colonne d'abattage) représente respectivement 98,5% et 0,9% des composés présents dans le gaz de réaction initial (rendement de récupération d'acide acrylique : 99,1%)

## Revendications

1. - Procédé de purification de l'acide (méth)acrylique obtenu par oxydation par voie catalytique ou par voie redox, d'un substrat gazeux constitué par du propane et/ou du propylène et/ou de l'acroléine dans le cas de la fabrication de l'acide acrylique, et par de l'isobutane et/ou de l'isobutène et/ou de l'alcool tertiobutylique et/ou de la méthacroléine dans le cas de la fabrication de l'acide méthacrylique, ledit mélange gazeux (1) étant principalement constitué par :
- du propane et/ou du propylène ou de l'isobutane et/ou de l'isobutène suivant que le substrat en comportait ;
- des produits d'oxydation ultime ;
- l'acide (méth) acrylique recherché ;
- de la (méth)acroléine ;
- de l'alcool tertiobutylique dans le cas de la fabrication l'acide méthacrylique ;
- de la vapeur d'eau ;
- de l'acide acétique avec, dans le cas de la fabrication de l'acide méthacrylique de l'acide acrylique comme sous-produit ; et
- des produits lourds de réactions secondaires,
suivant lequel on adresse le mélange gazeux de réaction (1) en pied d'une colonne d'absorption (C1) laquelle est alimentée en tête et à contre-courant par au moins un solvant lourd d'absorption hydrophobe, pour obtenir :
- en tête de la colonne (C1), un flux gazeux (7) constitué par :
- le propane et/ou le propylène ou l'isobutane et/ou l'isobutène, suivant que l'on fabrique l'acide acrylique ou l'acide méthacrylique, et les produits d'oxydation ultime du mélange (1) ;
- des quantités majeures d'eau et d'acide acétique dans le cas de la fabrication de l'acide acrylique, ou d'eau, d'acide acétique et d'acide acrylique dans le cas où l'on fabrique l'acide méthacrylique ; et
- la (métha)croléine ;
- en pied de ladite colonne (C1), un flux (4) constitué par :
- l'acide (méth)acrylique ;
- le ou les solvants lourds d'absorption ;
- les produits lourds de réactions secondaires ; et
- des quantités mineures d'acide acétique et d'eau, dans le cas de la fabrication de l'acide acrylique et d'acide acétique, d'acide acrylique et d'eau dans le cas de la fabrication de l'acide méthacrylique,
puis on adresse le flux (4) issu de la colonne (C1) à une colonne de séparation (C2) dans laquelle on conduit une séparation pour obtenir :
- en tête, un flux (5) constitué par les impuretés légères que l'on renvoie à la partie inférieure de la colonne d'absorption (C1) ; et
- en pied, un flux (6) constitué par :
- l'acide (méth)acrylique en solution dans le ou les solvants d'absorption ;
- une faible proportion d'acide acétique dans le cas de la fabrication de l'acide acrylique et d'acide acétique et d'acide acrylique dans le cas de la fabrication de l'acide méthacrylique ;
- les produits lourds des réactions secondaires ; et
- le ou les inhibiteurs de polymérisation,
**caractérisé par le fait que** l'on fait fonctionner la colonne (C1) avec un débit du solvant lourd qui est de 3 à 5,6 fois le débit de l'acide (méth) acrylique dans le mélange gazeux d'alimentation, et **par le fait que** l'on utilise, comme colonne de séparation (C2), une colonne de rectification que l'on fait fonctionner avec une alimentation en tête et sans reflux.

2. - Procédé selon la revendication 1, **caractérisé par le fait que** l'on fait fonctionner la colonne (C2) dans des conditions telles que son débit de tête rapporté au débit d'acide (méth)acrylique introduit dans la colonne d'absorption (C1) est compris entre 0,5/1 et 4/1.

3. - Procédé selon la revendication 2, **caractérisé par le fait que** l'on fait fonctionner la colonne (C2) dans des conditions telles que son débit de tête rapporté au débit d'acide (méth)acrylique introduit dans la colonne d'absorption (C1) est compris entre 2/1 et 3/1.

4. - Procédé selon l'une des revendications 1 à 3, **caractérisé par le fait que** l'on fait fonctionner la colonne (C1) avec un débit de solvant qui est de 3 à 4 fois le débit de l'acide (méth)acrylique dans le mélange gazeux d'alimentation.

5. - Procédé selon l'une des revendications 1 à 4, **caractérisé par le fait que** l'on utilise une colonne d'absorption (C1) comportant :
- dans sa partie inférieure, au moins une section de refroidissement (S1) équipée d'un système de recirculation, à travers un échangeur externe (E1) d'une partie (3) du flux (4) recueilli en partie inférieure de ladite ou desdites sections (S1) pour le renvoyer en tête desdites sections ; et
- dans sa partie supérieure, une section (S2) d'absorption et de rectification du mélange gazeux (1).

6. - Procédé selon la revendication 5, **caractérisé par le fait que** l'on utilise une section (S2) dont le nombre de plateaux théoriques est de 25 à 50, de préférence de 30 à 45.

7. - Procédé selon l'une des revendications 1 à 6, **caractérisé par** lé fait que l'on conduit l'absorption dans la colonne (C1) à la pression atmosphérique ou sous une pression proche de la pression atmosphérique et à une température d'introduction du ou des solvants de 20 à 80°C, de préférence de 30 à 60°C.

8. - Procédé selon l'une des revendications 1 à 7, **caractérisé par le fait que** l'on fait fonctionner la colonne (C1) à une température de pied de 50 à 120°C, notamment de 70 à 100°C.

9. - Procédé selon l'une des revendications 1 à 8, **caractérisé par le fait que** l'on fait fonctionner la colonne (C1) à une température des gaz en tête de 40 à 70°C, notamment de 50 à 60°C.

10. - Procédé selon l'une des revendications 1 à 9, **caractérisé par le fait que** l'on introduit les gaz de réaction à une température de 100°C à 200°C, notamment de 130°C à 180°C.

11. - Procédé selon l'une des revendications 1 à 10, **caractérisé par le fait que** l'on utilise un ou des solvants lourds d'absorption hydrophobes ayant un point d'ébullition sous pression atmosphérique supérieur à 200°C.

12. - Procédé selon la revendication 11, **caractérisé par le fait que** l'on utilise le ditolyléther comme solvant lourd hydrophobe.

13. - Procédé selon l'une des revendications 1 à 12, **caractérisé par le fait que** l'on alimente la colonne d'absorption (C1) par un ou des solvants purs et/ou par un ou des solvants provenant d'un recyclage d'un ou des flux obtenus dans des étapes ultérieures de purification.

14. - Procédé selon l'une des revendications 1 à 13, **caractérisé par le fait que** l'on conduit l'absorption dans la colonne (C1) en présence d'au moins un inhibiteur de polymérisation, choisi notamment parmi les dérivés phénoliques comme l'hydroquinone et ses dérivés tels que l'éther méthylique de l'hydroquinone, la phénothiazine et ses dérivés, tels que le bleu de méthylène, les quinones, telles que la benzoquinone, les thiocarbamates métalliques, tels que le dibutyldithiocarbamate de cuivre, les composés à groupements nitroso, tels que la N-nitroso-phénylhydroxylamine, les amines telles que les dérivés de la paraphénylènediamine, ou les composés N-oxyls, comme le 4-hydroxy-2,2,6,6-tétraméthylpipéridine-N-oxyl.

15. - Procédé selon l'une des revendications 1 à 14, **caractérisé par le fait que** l'on évacue le flux gazeux (7) issu de la tête de la colonne (C1), pour partie à la section de réaction, pour partie à une étape d'incinération ou de purge.

16. - Procédé selon l'une des revendications 1 à 14, **caractérisé par le fait que** l'on envoie le flux gazeux (7) issu de la tête de colonne (C1) en pied d'une section de condensation (S3) où ce mélange gazeux est mis en contact intime avec un courant liquide descendant (7') alimenté en tête de ladite section (S3) et constitué par le recyclage d'une partie du flux de pied de ladite section (S3) préalablement refroidie par un échangeur de chaleur externe (E4).

## Claims

1. Method for purifying (meth)acrylic acid obtained by catalytic or redox oxidation, of a gas substrate consisting of propane and/or propylene and/or acrolein in the case of the manufacture of acrylic acid, and of isobutane and/or isobutene and/or tert-butyl alcohol and/or methacrolein in the case of the manufacture of methacrylic acid, said gas mixture (1) mainly consisting of:
- propane and/or propylene or isobutane and/or isobutene if previously contained by the substrate;
- final oxidation products;
- the desired (meth)acrylic acid;
- (meth)acrolein;
- tertbutyl alcohol in the case of the manufacture of methacrylic acid;
- water vapor;
- acetic acid with, in the case of the manufacture of methacrylic acid, acrylic acid as a byproduct; and
- heavy products of side reactions,
according to which the reaction gas mixture (1) is sent to the bottom of an absorption column (C1) which is supplied at the top and in countercurrent with at least one heavy hydrophobic absorption solvent, to obtain:
- at the top of the column (C1) a gas stream (7) consisting of:
- propane and/or propylene or isobutane and/or isobutene, according to whether acrylic acid or methacrylic acid is manufactured, and the products of the final oxidation of the mixture (1);
- major quantities of water and acetic acid in the case of the manufacture of acrylic acid, or of water, acetic acid and acrylic acid in the case of the manufacture of methacrylic acid; and
- (meth)acrolein;
- at the bottom of said column (C1), a stream (4) consisting of:
- (meth)acrylic acid;
- the heavy absorption solvent or solvents;
- the heavy products of side reactions; and
- minor quantities of acetic acid and water, in the case of the manufacture of acrylic acid and acetic acid, acrylic acid and water in the case of the manufacture of methacrylic acid,
the stream (4) issuing from the column (C1) is then sent to a separation column (C2) in which a separation is carried out to obtain:
- at the top, a stream (5) consisting of light impurities which are sent to the bottom part of the absorption column (C1); and
- at the bottom, a stream (6) consisting of:
- (meth)acrylic acid in solution in the absorption solvent or solvents;
- a small proportion of acetic acid in the case of the manufacture of acrylic acid and acetic acid and of acrylic acid in the case of the manufacture of methacrylic acid;
- the heavy products of side reactions; and
- the polymerization inhibitor or inhibitors,
**characterized in that** the column (C1) is operated with a heavy solvent flow rate that is 3 to 5.6 times the flow rate of (meth)acrylic acid in the feed gas mixture, and **in that**, as a separation column (C2), a rectification column is used, which is operated with a flow feed and without reflux.

2. Method according to Claim 1, **characterized in that** the column (C2) is operated under conditions such that its distillate rate relative to the flow rate of (meth)acrylic acid introduced into the absorption column (C1) is between 0.5/1 and 4/1.

3. Method according to Claim 2, **characterized in that** the column (C2) is operated under conditions such that its distillate rate relative to the flow rate of (meth)acrylic acid introduced into the absorption column (C1) is between 2/1 and 3/1.

4. Method according to one of Claims 1 to 3, **characterized in that** the column (C1) is operated with a solvent flow rate that is 3 to 4 times the flow rate of (meth)acrylic acid in the feed gas mixture.

5. Method according to one of Claims 1 to 4, **characterized in that** an absorption column (C1) is used comprising:
- in its lower part, at least one cooling section (S1) equipped with a system for recirculating, via an external heat exchanger (E1), part (3) of the stream (4) collected in the lower part of said section or sections (S1) to send it to the flow of said sections; and
- in its upper part, a section (S2) for the absorption and rectification of the gas mixture (1).

6. Method according to Claim 5, **characterized in that** a section (S2) is used, in which the number of theoretical plates is 25 to 50, and preferably 30 to 45.

7. Method according to one of Claims 1 to 6, **characterized in that** the absorption is carried out in the column (C1) at atmospheric pressure or under a pressure close to atmospheric pressure, and at a solvent introduction temperature of 20 to 80°C, preferably 30 to 60°C.

8. Method according to one of Claims 1 to 7, **characterized in that** the column (C1) is operated at a bottom temperature of 50 to 120°C, particularly of 70 to 100°C.

9. Method according to one of Claims 1 to 8, **characterized in that** the column (C1) is operated at a overhead gas temperature of 40 to 70°C, particularly of 50 to 60°C.

10. Method according to one of Claims 1 to 9, **characterized in that** the reaction gases are introduced at a temperature of 100°C to 200°C, particularly 130°C to 180°C.

11. Method according to one of Claims 1 to 10, **characterized in that** one or more heavy hydrophobic absorption solvents are used, having a boiling point above 200°C at atmospheric pressure.

12. Method according to Claim 11, **characterized in that** ditolylether is used as a heavy hydrophobic solvent.

13. Method according to one of Claims 1 to 12, **characterized in that** the absorption column (C1) is fed with one or more pure solvents and/or with one or more solvents issuing from the recycling of one or more streams obtained from the subsequent purification steps.

14. Method according to one of Claims 1 to 13, **characterized in that** the absorption is carried out in the column (C1) in the presence of at least one polymerization inhibitor, selected in particular from phenolic derivatives such as hydroquinone and its derivatives such as methyl ether of hydroquinone, phenothiazine and its derivatives, such as methylene blue, quinones, such as benzoquinone, metal thiocarbamates, such as copper dibutyldithiocarbamate, compounds with nitroso groups, such as N-nitroso-phenylhydroxylamine, amines such as derivatives of paraphenylenediamine, or N-oxyl compounds, such as 4-hydroxy-2,2,6,6-tetramethylpiperidine-N-oxyl.

15. Method according to one of Claims 1 to 14, **characterized in that** the gas stream (7) issuing from the top of the column (C1) is removed, partly to the reaction section, and partly to an incineration or purge step.

16. Method according to one of Claims 1 to 14, **characterized in that** the gas stream (7) issuing from the top of the column (C1) is sent to the bottom of a condensation section (S3) where this gas mixture is placed in intimate contact with a descending liquid stream (7') supplied at the flow of said section (S3) and consisting of the recycling of part of the bottom stream of said section (S3) previously cooled by an external heat exchanger (E4).

## Patentansprüche

1. Verfahren zur Aufreinigung von (Meth)acrylsäure, die durch katalytische Oxidation oder durch Oxidation mittels einer Redox-Reaktion eines gasförmigen Substrats erhalten wurde, welches im Falle der Herstellung von Acrylsäure aus Propan und/oder Propylen und/oder Acrolein besteht und welches im Falle der Herstellung von Methacrylsäure aus Isobutan, und/oder Isobuten und/oder tert.-Butylalkohol und/oder Methacrolein besteht, wobei das gasförmige Gemisch (1) sich hauptsächlich aus folgenden Bestandteilen zusammensetzt:
- aus Propan und/oder Propylen oder aber Isobutan und/oder Isobuten, je nach Art des Substrates;
- aus den Produkten des letzten Oxidationsvorganges;
- aus der Zielverbindung (Meth)acrylsäure;
- aus (Meth)acrolein;
- aus tert.-Butylalkohol im Falle der Herstellung von Methacrylsäure;
- aus Wasserdampf;
- aus Essigsäure, wobei im Falle der Herstellung von Methacrylsäure darüber hinaus Acrylsäure als Nebenprodukt auftritt; und
- aus den schweren Produkten von Nebenreaktionen,
woraufhin die gasförmige Reaktionsmischung (1) in den Kolonnensumpf einer Absorptionskolonne (C1) eingeleitet wird, in welcher im Gegenstrom mindestens ein schwerflüchtiges hydrophobes Absorptionslösemittel vom Kolonnenkopf herabströmt, um Folgendes zu erhalten:
- am Kopf der Kolonne (C1) einen gasförmigen Stoffstrom (7), bestehend aus:
- Propan und/oder Propylen oder aber Isobutan und/oder Isobuten, je nachdem, ob Acrylsäure oder Methacrylsäure hergestellt wird, sowie aus den Produkten der letzten Oxidation des Gemisches (1);
- großen Mengen an Wasser und Essigsäure im Falle der Herstellung von Acrylsäure beziehungsweise an Wasser, Essigsäure und Acrylsäure im Falle der Herstellung von Methacrylsäure; und
- (Meth)acrolein
- am Sumpf der Kolonne (C1) einen Stoffstrom (4), bestehend aus:
- (Meth)acrylsäure;
- dem oder den schwerflüchtigen Absorptionslösemittel(n);
- den schweren Produkten von Nebenreaktionen; und
- kleinen Mengen an Essigsäure und Wasser im Falle der Herstellung von Acrylsäure beziehungsweise an Essigsäure, Acrylsäure und Wasser im Falle der Herstellung von Methacrylsäure,
woraufhin der Stromstrom (4), der aus der Kolonne (C1) stammt in eine Trennkolonne (C2) eingeleitet wird, in welcher eine Trennung durchgeführt wird, um Folgendes zu erhalten:
- am Kopf einen Stoffstrom (5), der aus den leichten Verunreinigungen besteht und der in den unteren Bereich der Absorptionskolonne (C1) zurückgeleitet wird, und
- am Sumpf einen Stoffstrom (6), bestehend aus:
- (Meth)acrylsäure, die in dem oder den Absorptionslösemittel(n) gelöst ist;
- einem geringen Anteil an Essigsäure im Falle der Herstellung von Acrylsäure beziehungsweise an Essigsäure und Acrylsäure im Falle der Herstellung von Methacrylsäure;
- den schweren Produkten von Nebenreaktionen; und
- dem oder den Polymerisationsinhibitoren,
**dadurch gekennzeichnet, dass** die Kolonne (C1) derart betrieben wird, dass die Flussrate des schwerflüchtigen Lösemittels 3 bis 5,6mal so hoch ist wie die Flussrate der (Meth)acrylsäure in der eingeleiteten gasförmigen Mischung und dass als Trennkolonne (C2) eine Rektifikationskolonne benutzt wird, die am Kopf gespeist und ohne Rückfluss betrieben wird.

2. Verfahren gemäß Anspruch 1, **dadurch gekennzeichnet, dass** die Kolonne (C2) unter solchen Bedingungen betrieben wird, dass das Verhältnis zwischen der Flussrate an ihrem Kopf und der Flussrate der (Meth)acrylsäure, welche in die Absorptionskolonne (C1) eingeleitet wird, zwischen 0,5/1 und 4/1 beträgt.

3. Verfahren gemäß Anspruch 2, **dadurch gekennzeichnet, dass** die Kolonne (C2) unter solchen Bedingungen betrieben wird, dass das Verhältnis zwischen der Flussrate an ihrem Kopf und der Flussrate der (Meth)acrylsäure, welche in die Absorptionskolonne (C1) eingeleitet wird, zwischen 2/1 und 3/1 beträgt.

4. Verfahren gemäß einem der Ansprüche 1 bis 3, **dadurch gekennzeichnet, dass** die Kolonne (C1) mit einer Lösemittelflussrate betrieben wird, welche 3 bis 4mal so hoch ist wie die Flussrate der (Meth)acrylsäure in der eingeleiteten gasförmigen Mischung.

5. Verfahren gemäß einem der Ansprüche 1 bis 4, **dadurch gekennzeichnet, dass** eine Absorptionskolonne (1) benutzt wird, die Folgendes aufweist:
- in ihrem unteren Bereich mindestens einen Kühlabschnitt (S1), der mit einem Kreislaufsystem zur Rückführung eines Teils (3) des Stoffstroms (4), der im unteren Bereich des/der Abschnitts/e (S1) anfällt, versehen ist, um den Stoffstrom in den Kopfbereich dieser Abschnitte zurückzuleiten, wobei die Rückführung über einen externen Tauscher (E1) verläuft; und
- in ihrem oberen Bereich einen Abschnitt (S2) zur Absorption und Rektifikation der gasförmigen Mischung (1).

6. Verfahren gemäß Anspruch 5, **dadurch gekennzeichnet, dass** ein Abschnitt (S2) zum Einsatz kommt, bei welchem die Anzahl theoretischer Böden 25 bis 50, vorzugsweise 30 bis 45 beträgt.

7. Verfahren gemäß einem der Ansprüche 1 bis 6, **dadurch gekennzeichnet, dass** die Absorption in der Kolonne (C1) bei Atmosphärendruck oder bei einem Druck, der dem Atmosphärendruck nahekommt, sowie bei einer Einleitungstemperatur des Lösemittels oder der Lösemittel durchgeführt wird, die 20 bis 80 °C, vorzugsweise 30 bis 60 °C beträgt.

8. Verfahren gemäß einem der Ansprüche 1 bis 7, **dadurch gekennzeichnet, dass** die Kolonne (C1) bei einer Sumpftemperatur zwischen 50 und 120 °C, insbesondere zwischen 70 und 100 °C betrieben wird.

9. Verfahren gemäß einem der Ansprüche 1 bis 8, **dadurch gekennzeichnet, dass** die Kolonne (C1) bei einer Kopftemperatur zwischen 40 und 70 °C, insbesondere zwischen 50 und 60 °C betrieben wird.

10. Verfahren gemäß einem der Ansprüche 1 bis 9, **dadurch gekennzeichnet, dass** die Reaktionsgase mit einer Temperatur zwischen 100 °C und 200 °C, insbesondere zwischen 130 °C und 180 °C eingeleitet werden.

11. Verfahren gemäß einem der Ansprüche 1 bis 10, **dadurch gekennzeichnet, dass** die schwerflüchtigen, hydrophoben Absorptionslösemittel einen Siedepunkt aufweisen, der bei Atmosphärendruck oberhalb von 200 °C liegt.

12. Verfahren gemäß Anspruch 11, **dadurch gekennzeichnet, dass** als schwerflüchtiges, hydrophobes Lösemittel Ditolylether verwendet wird.

13. Verfahren gemäß einem der Ansprüche 1 bis 12, **dadurch gekennzeichnet, dass** die Absorptionskolonne (C1) unter Einleitung eines oder mehrerer reiner Lösemittel und/oder eines oder mehrerer Lösemittel, die aus der Rückführung eines oder mehrerer Stoffströme aus späteren Aufreinigungsschritten stammen, betrieben wird.

14. Verfahren gemäß einem der Ansprüche 1 bis 13, **dadurch gekennzeichnet, dass** die Absorption in der Kolonne (C1) in Gegenwart mindestens eines Polymerisationsinhibitors durchgeführt wird, welcher insbesondere aus phenolischen Derivaten wie Hydrochinon und seinen Derivaten wie etwa Hydrochinonmethylether, aus Phenothiazin und seinen Derivaten wie etwa Methylenblau, aus Chinonen wie etwa Benzochinon, aus metallischen Thiocarbamaten wie etwa Kupferdibutyldithiocarbamat, aus Verbindungen mit Nitrosogruppen wie etwa N-Nitrosophenylhydroxylamin, aus Aminen wie etwa Derivaten des para-Phenylendiamins oder aus N-Oxylverbindungen wie etwa 4-Hydroxy-2,2,6,6-tetramethylpiperidin-N-oxyl gewählt wird.

15. Verfahren gemäß einem der Ansprüche 1 bis 14, **dadurch gekennzeichnet, dass** der gasförmige Stoffstrom (7), der aus dem Kopf der Kolonne (C1) stammt, teilweise im Reaktionsabschnitt und teilweise in einem Verbrennungs- oder Abblaseschritt entfernt wird.

16. Verfahren gemäß einem der Ansprüche 1 bis 14, **dadurch gekennzeichnet, dass** der gasförmige Stoffstrom (7), der aus dem Kopf der Kolonne (C1) stammt, dem Sumpf eines Kondensationsabschnittes (S3) zugeführt wird, in welchem dieses gasförmige Gemisch mit einem herabfließenden Flüssigkeitsstrom (7') innig in Kontakt gebracht wird, wobei der Flüssigkeitsstrom am Kopf dieses Abschnitts (S3) eingeleitet wird und aus einem Teil des Stoffstroms am Sumpf des Abschnitts (S3) besteht, wobei dieser Teil vor seiner Rückführung mittels eines externen Wärmetauschers (E4) abgekühlt wird.
